# EUROPEAN PATENT SPECIFICATION

(11) **EP 0 329 264 B1**
(45) Date of publication and mention of the grant of the patent: **23.03.1994**
(21) Application number: 89201017.4
(22) Date of filing: 14.06.1984
(51) Int. Cl.: A61K 39/39, A61K 39/215

(54) **Veterinary vaccines**
Tierärztliche Impfstoffe
Vaccins vétérinaires

(30) Priority: 15.06.1983 US 504434; 07.06.1984 US 618638
(43) Date of publication of application: 23.08.1989
(62) Divisional of application: 84902632.3
(73) Proprietor: AMERICAN HOME PRODUCTS CORPORATION, Madison, New Jersey 07940-0874 (US)
(72) Inventor: Acree, William Marshall, Temple Texas 76501 (US); Edwards, Bobby, Temple Texas 76501 (US); Black, John Wesley, Mitton Tennessee 37118 (US)
(74) Representative: Brown, Keith John Symons

(56) References cited:
- CANINI PRACTICE, vol. 7, no. 4, July-August 1980, pages 22-35; M. APPEL et al.: "Canine viral enteritis"
- Principles of Bacteriology, Virology and Immunity, Topley & Wilson, 6th ed., 1975, p. 1459. 1464

## Description

This invention relates to veterinary vaccines and, in particular to veterinary vaccines containing adjuvants.

According to the present invention there is provided a veterinary vaccine composition comprising viral antigen, a pharmaceutically acceptable carrier, and an adjuvant which is at least one member selected from ethylene maleic anhydride and a copolymer of styrene with a mixture of acrylic acid and methacrylic acid.

The invention also provides a process for preparing a veterinary vaccine composition which comprises bringing into association a viral antigen, a pharmaceutically acceptable carrier and an adjuvant which is at least one member selected from ethylene maleic anhydride and a copolymer of styrene with a mixture of acrylic acid and methacrylic acid.

Preferably the veterinary vaccine is a canine coronavirus vaccine. Our copending European application 84902632.3 (publication number 0145783), from which the present application is divided, discloses a canine coronavirus vaccine and its method of preparation. The above-mentioned European application is hereinbefore referred to as the "parent application".

The parent application particularly provides a vaccine composition comprising the avirulent antigenic product produced by either
(a) attenuating live canine coronavirus by passage through cells of feline origin such that when administered to a dog by injection the attenuated live virus selectively infects the intestinal epithelium, or
(b) inactivating feline or canine cell propagated canine coronavirus,
the avirulent antigenic product being present in an amount effective to protect a dog from infection by virulent canine coronavirus;
and a non-toxic pharmaceutically acceptable carrier.

The original isolation of the virulent CCV was made from a dog which died of gastroenteritis. The isolate was given the designation TN-449. Virus detection methods including electron microscopy indicated CCV as the only virus present in the specimens taken from the dog. This virus isolate was then placed in CRFK cell cultures for propagation and attenuation purposes. A low virus inoculum was placed in CRFK cell cultures which became confluent in 24 hours or less. A virus to cell ratio of about 1 to 3,000 was utilized. The low virus input requires an increased number of virus multiplications which aids in the attenuation process. The virus passaging took about two months. Twelve consecutive cell passages were performed in a heterologous cell culture such as CRFK to achieve attenuation. Dogs were inoculated with various dose levels of passage 12 material. Dose levels of 7.5 log₁₀, 5.5 log₁₀, 3.5 log₁₀ and 1.5 log₁₀ were used parenterally in dogs. A non-inoculated dog was held as a contact control dog. These animals were observed following vaccination. The dogs remained healthy with no symptoms of gastroenteritis being observed. Post inoculation serology verify the virus inoculum had replicated in the dogs. This demonstrated the attenuation of the TN-449 virus. An additional virus passage of the TN-449 was made to establish the Master Seed Virus. The TN-449 culture has been deposited with the American Type Culture Collection in Rockville, Maryland and has been given ATCC Deposit No VR 2068.

The new attenuated (ie modified) live canine coronavirus of the parent application has three distinguishing characteristics which make it useful as a vaccine virus (1) the attenuated CCV is fully adapted to growth and reproduction in cell culture, especially cells of feline or canine origin, (2) the attenuated CCV does not produce disease when administered to a dog and (3) when administered by injection, eg subcutaneously, parenterally, etc, the virus selectively infects intestinal epithelium. Because of these unique characteristics of the attenuated CCV localized immunity in the intestines is produced when the virus is administered to dogs.

These unique characteristics appear to be due to the method used to attenuate the virus. Attenuated live CCV suitable for use in the parent invention is prepared by passing a virulent CCV strain in cells of feline origin at least eight times at a low virus to cell ratio of 1:1,000-10,000, preferably 1:1,500-7,500 wherein the virus particles are measured by the TCID₅₀ method (Tissue Culture Infective Dose).

Passing the virus at a low virus to cell ratio speeds up attenuation and allows the cells to become attenuated with a minimum number of passages. The low virus to cell ratio also acts to genetically select only those cells which are fully adapted to cell culture, thereby producing an attenuated virus strain which is useful in a vaccine. For attenuation purposes, feline cells should be used and canine cells should be avoided. The CCV appears to become attenuated easily in feline cells, however, if canine cells are used a possibility exists that the virus would revert back to the virulent state. Thus, when the attenuated CCV is grown for production purposes, more than one or two passages in canine cells should be avoided. The cells should be passed through the feline cells at least eight times usually 8 to 60 passages and preferably 8 to 25 passages.

The CCV vaccine composition according to the parent application includes an attenuated live virus having a titer of greater than 2.5 logs₁₀ of virus particles/ml (or dose) preferably at least 3.0 logs of virus particles/ml (or dose) and more preferably greater than 3.3 logs of virus particles/ml (or dose) as measured by the FAID₅₀ (Fluorescent Antibody Infectious Dose) method (King et al, Can. Journal of Comparative Medicine & Vet. Science, 29, pp. 85-89 (1965)). Titers as high as 5 to 7 logs FAID₅₀ have been obtained. The CCV vaccine composition includes an attenuated live CCV and also may contain an attenuated live or killed CPV virus. In addition, the vaccine may contain additional attenuated live virus or killed viruses such as Canine Distemper virus, Canine Parainfluenza virus, Canine Adenovirus I, Canine Adenovirus II and Canine Rotavirus. The attenuated live CCV virus produces a complete immunological response to the virus infection. However, the infection produces a fever about 0.5 to 1.0°F (about 0.28 to 0.55° C) lower than the virulent strain of CCV. The vaccine composition contains a sufficient amount of the attenuated live virus to produce an immunological response in a dog and a non-toxic pharmaceutically acceptable sterile carrier or diluent such as a sterile saline solution. Preferably the carrier or diluent is suitable for parenteral injection, however, the carrier or diluent may also be of a type which is suitable for use as a nasal spray.

The method for propagating the CCV in mammalian cells according to the parent application includes the steps of inoculating mammalian tissue culture cells with CCV, cultivating the cells into a one hundred percent (100%) confluent tight monolayer before said inoculating step or within 24 hours of said inoculating step, harvesting the cells between 24 and 96 hours after inoculation and collecting the propagated virus from the harvested cells.

The mammalian tissue culture cells are inoculated with CCV at a CCV virus (measured by the FAID₅₀ method) to cell ratio of 1:500 to 1:1, preferably 1:100 to 1:5, more preferably 1:75 to 1:10. The mammalian tissue culture cells include but are not limited to Crandall Feline Kidney Cells (CRFK), Wood's Feline Cell Line (FC), a Dog Kidney Cell Line (DK), Madin Darby Canine Kidney (MDCK) and the canine A72 cell line. The CCV is added to a suspension of the cells or is applied to a monolayer of the cells. The amount of cells and virus should be such that a confluent tight monolayer of the cells will form within 12-48, preferably 24-36, hours after inoculation. Optimally, at the time of inoculation the cells should be present in the growth vessel in an amount sufficient to form a monolayer of cells of at least 100,000 to 1,000,000 cells per square centimeter (cm²) within about 12-48 hours after inoculation, preferably within 24 hours after inoculation. However, a lesser number of cells may be used. Preferably the cells are present in an amount sufficient to form between 150,000 to 500,000 cells/cm² within 12-48 hours, preferably within 24 hours. The virus is absorbed on the cells for at least 60 minutes but usually less than 300 minutes, preferably between 90 and 240 minutes at 28 to 40° C, preferably 35 to 38° C.

Harvestable virus titers of at least about 1,000 and usually at least about 2,000 as measured by the FAID₅₀ method can be obtained within 18 hours after inoculation, however, in some circumstances it may take up to 120 hours or longer to obtain maximum virus titers. Maximum virus titers are usually obtained within about 24 to 96 hours after inoculation. The cell monolayer is removed either by freeze-thawing or by enzymatic action to increase the viral content of the harvested fluids. The harvested fluids are then combined with a virus stabilizer such as sucrose phosphate glutamate (SPG), sucrose phosphate glutamate albumin (SPGA) or NZ amine-gelatin for final product filling or frozen in bulk for later thawing and bulking with virus stabilizer. Alternatively, the harvested fluids may be inactivated.

Isolates of Canine Coronavirus such as the I-171 strain (ATCC VR-809), the TN-449 (ATCC VR-2068) or any of three canine coronaviruses available from Cornell University (K378-8, S378-6 and A76 strains) may be used for manufacturing an inactivated CCV vaccine. The inactivated CCV vaccine composition includes one or more isolates of an inactivated canine coronavirus having pre-inactivation virus titers of greater than 4.0 logs of virus particles/ml (or dose) and preferably greater than 5.0 logs of virus particles/ml (or dose) as measured by the FAID₅₀ (Fluorescent Antibody Infectious Dose) method (King et al, Can. Journal of Comparative Medicine and Vet Science 29 pp 85-89, 1965). The typical dose amount is one milliliter.

Inactivated CCV fluids may also be concentrated by any number of available techniques such as an Amicon concentrating device, Pellicon (Millipore) concentration device, precipitation techniques, such as ammonium chloride, concentration with Carbowax liquid or wax in conjunction with dialysis tubing, or adjuvant concentration techniques, such as with aluminium phosphate.

Titers as high as 5 to 7 logs FAID₅₀ have been obtained. The inactivated CCV vaccine composition includes one or more inactivated canine coronaviruses and also may contain an attenuated modified live or killed canine parvovirus (CPV). In addition, the vaccine may contain in any combination or singularly, additional attenuated modified live viruses or killed viruses such as Canine Distemper virus, Canine Parainfluenza virus, Canine Adenovirus I, Canine Adenovirus II, Canine Rotavirus, Measles virus, Canine Calicivirus and Canine Herpesvirus. The inactivated CCV vaccine produces a complete immunological response to the virus infection.

The vaccine composition according to the parent application comprises a sufficient amount of the canine corona-virus antigen to produce an immunological response in a dog and a non-toxic pharmaceutically acceptable adjuvant or adjuvant combination.

A further aspect of the invention of the parent application concerns the route of administration. The induction of humoral-systemic protection as measured by serum neutralizing antibody levels as well as the induction of local immunity in the intestinal tract as an important feature of the invention. The oral-intranasal routes of administration should elicit both types of protection. However, the general marketplace acceptance of this route of administration is not good and, therefore, the use and subsequent protection afforded by a CVV vaccine would not be realized. An attenuated live virus vaccine which is parenterally administered will provide humoral and localized immunity. The parenteral vaccination with an inactivated CCV vaccine will also induce both humoral immunity and localized immunity. The term parenteral vaccination is intended to include subcutaneous and intramuscular inoculation routes.

The attenuated vaccine is usually parenterally administered in a dose of at least 2.5 logs of virus particles per dose, preferably at least 1,000 virus particles (3 logs of virus particles) per dose, more preferably at least 3.3 logs of virus particles per dose.

The inactivated vaccine is preferably parenterally administered in a dose of at least 10,000 inactivated virus particles (4 logs of virus particles) per dose, preferably at least 5.0 logs of virus particles per dose. The vaccine may be administered in either a one or two dose regimen. The second dose should be no more than six weeks after the first. Preferably the second dose is administered two to three weeks after the first dose.

Another aspect of invention of the parent application is the inactivation of the canine coronavirus. CCV fluids can be inactivated with a number of inactivating agents such as, but not limited to, binary ethylenimine, acetyl ethylenimine, beta-propiolactone, formalin, phenol, ultraviolet radiation or gamma irradiation.

Beta-propiolactone at concentrations of 0.01 to 0.5% are preferably used. Concentrations of beta-propiolactone of 0.05% to 0.2% being most frequently used. The inactivating agent is added to virus fluids contained in the propagating vessels, fluids which have been harvested from the propagating vessel and combined or virus fluids which have been harvested from the propagating vessels, combined, stored, frozen and then thawed.

Beta-propiolactone is added to the virus fluids, with the adverse shift in pH to acidity being controlled with sodium hydroxide or sodium becarbonate solution. The combined inactivating agent-virus fluids are incubated at temperatures from 4°C to 37°C. Incubation times of 24 to 72 hours are used.

Another inactivant used is binary ethylenimine. Equal volumes of a 0.2 molar bromoethylamine hydrobromide solution and a 0.4 molar sodium hydroxide solution are mixed and incubated at about 37° C for 60 minutes. The resulting cyclized inactivant is binary ethylenimine, which is added to the virus fluids at 0.5 to 4 percent, volume to volume. The inactivating virus fluids are held from about 4°-37° C for 24 to 72 hours with periodic agitation.

A feature of the present invention is the adjuvant system. Individual or a combination of adjuvants may be utilized to inhance the immune response. Examples of adjuvants according to the invention are ethylene maleic anhydride and Neocryl A640. Other adjuvants, such as aluminium phosphate and aluminium hydroxide may be used in combination with the adjuvants of the present invention. Neocryl is a trade name for a latex emulsion of a copolymer of styrene and a mixture of acrylic acid and methacrylic acid. Neocryl A640 is an uncoalesced aqueous acrylic copolymer with styrene, having pH 7.5, viscosity 100cps (Brookfield 25° C), weight per gallon is 8.6 pounds as supplied containing 40 percent solids by weight and 38 percent solids by volume. The numeral A640 denotes a grade thereof. Other useful Neocryl grades are 520, 625 and 966. The term "CSMA" will be used hereinafter to refer to a copolymer of styrene with a mixture of acrylic acid and methacrylic acid.

Ethylene maleic anhydride (EMA) prepared at a 5 percent weight/per vol. concentration in water added to the inactivated virus fluids at 0.01 percent to 6 percent volume to volume concentration. The pH of the resulting fluids is adjusted to 7.7 by addition of 1 normal sodium hydroxide.

CSMA prepared in a 50 percent volume per volume suspension in water is added to the inactivated CCV fluids from 0.1 percent to 10 percent volume. Usually there is no need for a pH adjustment as the CSMA is of a neutral pH.

Combining of two or more adjuvants may be accomplished as in the following, but are not limited to this combination. Ethylene maleic anhydride as described is added at 0.01 percent to 6 percent volume to volume to the inactivated virus fluids. The mixture is adjusted to a 7.1 to 7.7 pH by addition of 1 normal sodium hydroxide. The addition of 0.01 percent to 10 percent of the CSMA, as described, is accomplished with no further pH adjustments being required.

In order more clearly to disclose the nature of the present invention, specific examples are hereinafter given. Some of the examples exemplify the invention disclosed in the parent application. In the examples, all temperatures are stated in degrees centigrade, logs means logs to the base 10, and the following abbreviations are used: "g." for grams, "mcg." for micrograms, "ml." for milliliters, "min." for minutes, and "hr." for hours.

### EXAMPLE 1

### This example illustrates vaccine production.

The production strain was originally isolated from a dog which had died of CCV enteritis. The virus was serially propagated in Crandall Feline Kidney Tissue (CRFK) culture. Upon receipt the virus was designated CCV-MSV and passaged once to Master Seed CCV-MSV(X). The CCV-MSV (Canine Corona Virus-Master Seed Virus) culture has been deposited with the American Type Culture Collection in Rockville, Maryland and given ATCC Deposit No VR 2068. In vaccine production the cell cultures were grown in dynamic cultures. In some preliminary runs, static cultures were used. Cell cultures were grown in mineral essential media (MEM) supplemented with vitamins, non-essential amino acids, sodium pyruvate, sodium bicarbonate and L-glutamine. The amount of 30 mcg/ml. of gentamicin was added as a preservative. A 5-10 percent by weight concentration of bovine serum was added for cell growth. The serum concentration was reduced to not greater than 1 percent for a maintenance medium. Trypsin was added at a concentration of 0.05 ml./liter of medium to promote virus infectivity.

Confluent cultures of CRFK cells were trypsinized and planted into roller cultures so that a density of 150,000 cells per cm² was obtained after 24 hours. Cultures were grown at 28 to 40° C, preferably 35-38° C. The growth media was removed.

The production seed virus was thawed in cool running water. Sufficient virus was added to achieve a minimum multiplicity of infection (MOI) ratio of 1:100. A 2000 cm² bottle contained 300-500 million cells. At a 1:50 MOI 300 x 10⁶ divided by 50 yielded a minimal infective virus inoculum of 6 x 10⁶ or 10^{6.8}FAID₅₀ per roller. Virus seeds and product harvests yielded 10^{5.5} to 10^{7.0} FAID ₅₀ per ml. Thus 20 ml. of undiluted to 1 ml. of undiluted seed was usually used per bottle. The seed was brought up to a volume of 50 ml. per roller bottle with culture medium. The virus was allowed to adsorb on monolayers or in suspension for 2 hours at 35 to 38° C.

A specific example used a seed titered at 10^{5.7}FAID₅₀ per ml. with 15 ml. per roller used as the inoculum. Thirty two roller bottles were inoculated as described. The bottles were refed with 2 liters of MEM at 1 percent FCS and 0.5 ml. of trypsin. The fluids were harvested along with the cellular material 48 hours after infection, dispensed and frozen at -40° C or lower.

The bottles yielded 66.5 liters of virus fluids. The titer of the fluids was 10^{5.0}FAID₅₀ₛ per ml. The material was later thawed and blended with media and sucrose phosphae glutamate albumin (SPGA) stabilizer and lyophilized.

### EXAMPLE 2

### This example illustrates intestinal propagation through parenteral inoculation.

The purpose of the study was to determine whether the CCV vaccine would locate the replicate in the intestinal tract when administered intranasally or intramuscularly to healthy susceptbile dogs.

Four dogs were divided into two groups, isolated, and bled prior to vacination. Dogs Nos. 63 and 64 were vaccinated intramuscularly with a 10 ml. dose (10^{6.3} FAID₅₀/dose) of ccv (x + 1) seed (seed prepared by one additional passage from the master seed). The two other dogs Nos. 62 ad 65 were vaccinated intranasally with a 2 ml dose (10^{5.6} FAID₅₀/dose) of the same CCV seed. On day 5 after vaccination the dogs were bled, swabbed for fecal samples for virus isolation in CRFK cell culture, and euthanized Tissue scrapings from the trachea, duodenum, jejunum and cecum were placed on slides. These impression smears were then subjected to observation for virus replication by the direct fluorescent antibody technique.

### Fecal Virus Isolation

No Coronavirus could be isolated from the feces.

### Direct FA Staining of Impression Slides

The results of fluorescent antibody staining of tissue impression slides are shown in Table 2-1.

**TABLE 2-1**

| CCV Direct FA of Impression Slides | | | | | | | | |
|---|---|---|---|---|---|---|---|---|
| Dog No. | Virus Input (FAID)₅₀ /dose | Vaccination Route | CCV+ slides/No. of slides taken | | | | CCV+Slides/Total Slides | CCV Positive (%) |
| | | | Trachea | Duodenum | Jejunum | Cecum | | |
| 63 | 10^{6.3} | IM | NA | 10/12 | 4/7 | 3/5 | 17/24 | 71 |
| 64 | 10^{6.3} | IM | NA | 3/6 | 4/5 | 4/5 | 11/16 | 69 |
| 62 | 10^{5.7} | IN | 3/4 | 2/2 | 2/2 | 2/2 | 6/6 | 100 |
| 65 | 10^{5.7} | IN | 2/2 | 2/2 | 4/4 | 5/5 | 11/11 | 100 |

One hundred percent of the intestinal impression slides taken from the intranasally inoculated dogs were positive for virus replication, while seventy percent of the intestinal impresson slides were positve for virus replication from the dogs inoculated intramuscularly.

Virus replication occured in all three sections of the intestinal tract. Virus replication was also observed in the trachea of the dogs vaccinated intranasally.

The degree of virus involvement in the intestine of intranasally inoculated animals and the fact that the trachea also exhibited virus replication may indicate this route of inoculation as the natural portal of entry for infection.

### Conclusion

Virus replication in the intestinal tract can be achieved 5 days afer administering high dose levels of virus either intramuscularly or intranasally.

### EXAMPLE 3

### This example illustrates low virus inoculum parenterally administered for intestinal replication.

The purpose of this study was to determine whether the CCV vaccine when administered intramuscularly in a low dose level would locate and replicate in the intestinal tract.

Two dogs Nos. 60 and 68 were vaccinated intramuscularly with 1 ml. of the CCV-MSV (x + 3) seed diluted to 10^{3.0}FAID₅₀/ml. On day 5 after vaccination the dogs were bled and euthanized. Impression smears from villi scrapings were made from the duodenum, jejunum and cecum for direct FA observation to detect virus replication. These smears were processed and stained with CCV specifc FITC (fluoroisothiocyanate) bound conjugate and examined via FA.

### Direct CCV FA from Villi Scrapings

Recovery of positive CCV from the villi scraping slides are shown in Table 3-1.

**TABLE 3-1**

| CCV Direct FA of Villi | | | | | | | |
|---|---|---|---|---|---|---|---|
| Dog No. | Virus Input | Vaccination Route | CCV + slides/No. of slides taken | | | CCV+Slides /Total Slides | CCV Positive (%) |
| | | | Duodenum | Jejunum | Cecum | | |
| 60 | 10^{3.0} | IM | 6/7 | 5/7 | 6/6 | 17/20 | 85 |
| 68 | 10^{3.0} | IM | 4/6 | 5/6 | 4/6 | 13/18 | 72 |
| | | | | | | | A̅v̅g̅.̅ %̅=̅7̅9̅ |

CCV replication was found in all areas of the intestinal tract of both dogs 5 days following the intramuscular administration of 3 logs of virus. An average of 79% of the samples observed were positve for virus replication. This percentage compares favorably with the 70% positive sample obtained with dogs administered with 5.6 logs of virus in another study.

CCV replication can be demonstrated in the intestinal tract of susceptible dogs vaccinated parenterally with 10^{3.0}FAID₅₀/dose of CCV vaccine 5 days after vaccination.

### EXAMPLE 4

### Preliminary Vaccine and Challenge Evaluation

The purpose of this study was to evaluate a Canine Corona Challenge Virus and to determine if the vaccine virus will prevent any symptoms induced by the challenge virus and reduce or prevent intestinal infection by the challenge virus.

Two Canine Corona virus-susceptible dogs, Nos. 61 and 66, were bled and vaccinated intramuscularly with 3 logs of CCV MSV (x + 3) seed. These animals were held in isolation until the time of challenge.

At 21 days after vaccination the two CCV vaccinates along with the four CCV susceptible dogs Nos. 75, 79, 74 ad 76 were bled, anesthetized and challenged with 5 ml./dog of CCV challenge virus. Each dog received 2 ml. orally and 3 ml. intranasally. Each dog received 10^{5.7}FAID₅₀ of challenge virus.

Dogs Nos. 74 ad 76 were monitored for 7 days after challenge for temperature response, WBC (white blood cell) count, lymphocyte count response and other symptoms. At 5 days after challenge the two CCV vaccinates Nos. 61 and 66 along with the two challenge control dogs Nos. 75 and 79 were euthanized. Intestinal scraping impression smears were made of the duodenum, jejunum and cecum of each dog. Fluorescent antibody staining was performed on these smears to determine the extent of viral replication in the intestinal tract.

**TABLE 4-1**

| CCV SN Testing | | | |
|---|---|---|---|
| Dog No. | Group | CCV Serum Neutralizing (SN) Antibody Titer | |
| | | Pre-vaccination | Pre-Challenge |
| 61 | V* | < 1:2 | 1:1122 |
| 66 | V | < 1:2 | 1:1122 |
| 75 | C** | | < 1:2 |
| 79 | C | | < 1:2 |
| 74 | C | | < 1:2 |
| 76 | C | | < 1:2 |

| | | | |
|---|---|---|---|
| * Vaccinate | | | |
| ** Challenge Control | | | |

Both vaccinates were seronegative prior to vaccination and serocoverted to a 1:1122 level 21 days after vaccination.

An average of 95.6 percent of all intestinal samples observed in the controls demonstrated a 4(+) virus replication level. None of the vaccinates demonstrated a 4(+) level of virus infection in the intestinal samples. Therefore a 100% reduction in the 4(+) level of infection was achieved.

A total of 17.4 percent of the vaccinate samples were positive with a 1(+) level of infection whereas a total of 100% of the challenge control samples were positive (95.6% at 4+, 4.4% at 1+). An overall percent reduction in intestinal infection of 82.6% was achieved in the vaccinates.

Further analysis of this data can be performed which gives significance to the 4+ level of infection in comparison with a 1+ level of infection. This analysis can be found in Table 4-2.

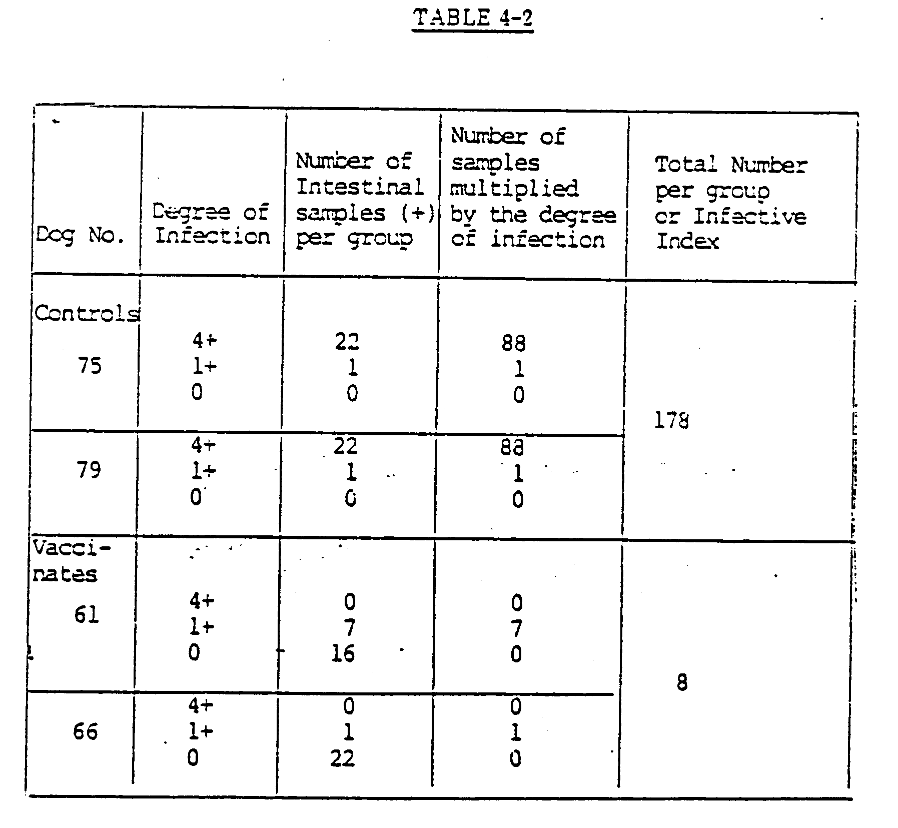

### CCV Challenge Evaluation Results and Discussion

### 1. Temperature Response

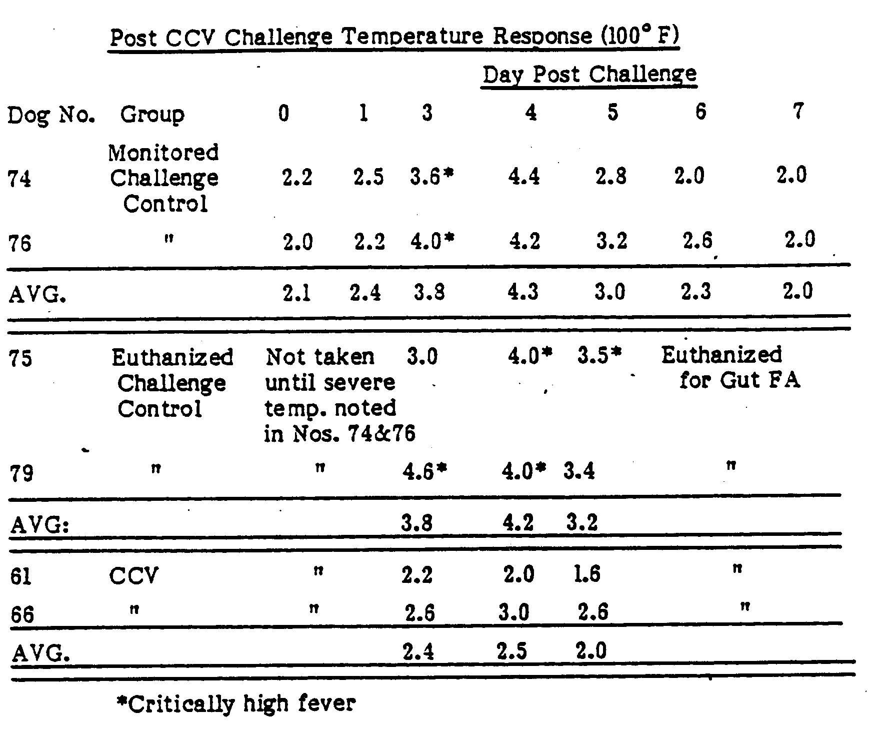

The CCV challenge produced a critically high fever (103.5° F) in the CCV susceptible challenge control dogs for day 3, 4, 5 respectively. All of the four challenge control dogs expressed temperatures of 103.5F for two consecutive days. Dogs 74 and 76 averaged temperature of 103.8F and 104.3F on day 3 after challenge and 104° F on day 4 after challenge. Dog 75 had a delayed temperature response of 104° F and 103.5° F on days 4 ad 5 after challenge.

The CCV vaccinated dogs displayed no temperature over 103° F for the 3 days they were monitored during the critical fever period. The CCV challenge produced a significant febrile response in CCV susceptible dogs and the CCV vaccine prevented this temperature increase.

### 2. WBC Response

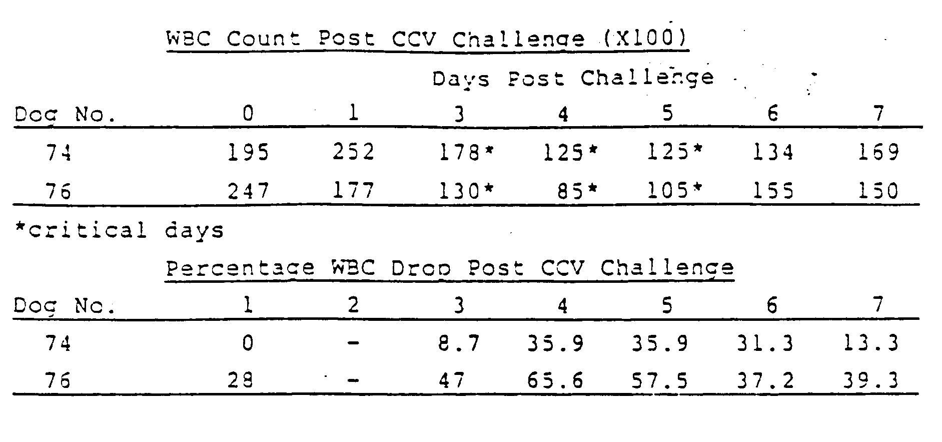

The critical period for a WBC drop following CCV challenge was on days 3, 4 and 5. This data indicates WBC monitoring may be a critical parameter for the Canine Coronavirus disease syndrome.

### 3. Lymphocyte Count

| Lymphocyte Count Post CCV Challenge (X100) | | | | | | | |
|---|---|---|---|---|---|---|---|
| Dog. No. | Days Post Challenge | | | | | | |
| | 0 | 1 | 3 | 4 | 5 | 6 | 7 |
| 74 | 82 | 103 | 43* | 39* | 31* | 56 | 61 |
| 76 | 131 | 58* | 46* | 29* | 29* | 68 | 65 |

| | | | | | | | |
|---|---|---|---|---|---|---|---|
| *critical days | | | | | | | |

A lymphopenia developed in the CCV challenge control dogs beginning on day 1 with dog 76 and day 3 with dog 74. The lymphopenia continued through day 5 after challenge. Further analysis of the percent lymphocyte drop is listed below:

| Percentage Lymphocyte Drop Post CCV Challenge | | | | | | |
|---|---|---|---|---|---|---|
| Dog. No. | Days Post Challenge | | | | | |
| | 1 | 3 | 4 | 5 | 6 | 7 |
| 74 | - | 48 | 52* | 62* | 32 | 26 |
| 76 | 36* | 65* | 78* | 78* | 48 | 50 |

| | | | | | | |
|---|---|---|---|---|---|---|
| *critical days | | | | | | |

Lymphopenia with over a 50% reduction in lymphocytes occurred in 6 of 12 observations of the challenge control dogs with the most severe occurrences on day's 4 and 5 after challenge. Dog 74 had greater than 50% lymphopenia on days 4 and 5 after challenge. Dog 76 displayed a lymphopenia over 50% on days 3, 4, 5 and 7 after challenge and had the largest drop of 78% on days 4 and 5 after challenge.

### 4. Symptoms

| Symptoms Post CCV Challenge | | | | | | | |
|---|---|---|---|---|---|---|---|
| Dog No. | Days Post Challenge | | | | | | |
| | 0 | 1 | 3 | 4 | 5 | 6 | 7 |
| 74 | None | None | Nasal Discharge Anorexia Dehydration Feces Dry | Slight Nasal Discharge Slight Ocular Discharge Anorexia Dehydration Feces Wet | Soft Stool | Slight Nasal Discharge | Slight Nasal and Ocular Discharge |
| 76 | None | None | Same as Above | Same as Above | Slight Nasal Discharge Feces Soft | Same as Above | Same as Above |

Symptoms of CCV infection began on day 3 after challenge and ran concurrent with the febrile response and lymphopenia. Symptoms or nasal discharge, anorexia, dehydration, and ocular discharge continue through day 7 after challenge, the last day of monitoring. The CCV challenge produced significant CCV symptoms in CCV susceptible dogs.

### Evaluation of Virus Replication in the Intestinal Tract of Vaccinates and Controls 5 Days After Challenge

A total of 8 locations were selected in the trachea for impression slide preparations and 23 locations were selected along the intestinal tract for evaluation. Intestinal scrapings were placed on slides which were acetone fixed and stained with specific Canine Coronavirus FA conjugate. The degree of replication was evaluated as to a 4+ infection, 1+ infection or no virus present. The results of these evaluations are shown in Tables 4-3 and 4-4.

**TABLE 4-3**

| Sample Evaluation for Canine Coronavirus Replication | | | | | | | |
|---|---|---|---|---|---|---|---|
| Group | Dog No, | Degree of FA+ | No. of Slides | | | | |
| | | | Trachea | Duodenum | Jejenum | Cecum | TOTAL |
| Challenge control | 75 | 4+ | 0 | 6 | 8 | 8 | 22 |
| | | 1+ | 0 | 1 | 0 | 0 | 1 |
| | | No CCV | 8 | 0 | 0 | 0 | 8 |
| Challenge control | 79 | 4+ | 0 | 6 | 8 | 8 | 22 |
| | | 1+ | 0 | 1 | 0 | 0 | 1 |
| | | No CCV | 8 | 0 | 0 | 0 | 8 |
| Vaccinate | 61 | 4+ | 0 | 0 | 0 | 0 | 0 |
| | | 1+ | 0 | 2 | 4 | 1 | 7 |
| | | No CCV | 8 | 5 | 4 | 7 | 24 |
| Vaccinate | 66 | 4+ | 0 | 0 | 0 | 0 | 0 |
| | | 1+ | 0 | 0 | 0 | 1 | 1 |
| | | No CCV | 8 | 7 | 8 | 7 | 30 |

No Trachea virus replication was noted in any of the dogs. Further evaluation of the percent of positive samples will exclude the trachea samples.

### EXAMPLE 4.4

| Dogs | Percent of samples FA(+) per degree of infection in the intestinal tract | | | Average Total % per test group | |
|---|---|---|---|---|---|
| | 4+ | 1+ | 0 | (+) | (-) |
| 75 | 95.6 | 4.4 | 0 | | |
| 79 | 95.6 | 4.4 | 0 | | |
| Control Average | 95.6 | 4.4 | 0 | 100 | 0 |
| 61 | 0 | 30.4 | 69.6 | | |
| 66 | 0 | 4.4 | 95.6 | | |
| Vaccinate Average | 0 | 17.4 | 82.4 | 17.4 | 82.4 |

An infective index level of 178 for the controls versus 8 from the vaccinates indicates a reduction in infection of 95.5%.

The study thus establishes that the CCV challenge produced severe measurable clinical symtoms of CCV disease and the CCV challenge can be observed in the gut. The CCV vaccine lowers the degree of infection in the vaccinated dog's gut and eliminates the clinical symptoms and fever after CCV challenge when compared with CCV susceptible challenge control dogs.

### EXAMPLE 5

### This example illustrates Vaccine Efficacy.

Pups were proven seronegative to CCV by a serum neutralization test via F.A. Twenty-two of the animals were vaccinated with a minimal dose of vaccine. One half of the group was given the vaccine subcutaneously and the other half of the group was administered vaccine intramuscularly. The vaccinated dogs then were housed individually but kept in the same area along with three more pups, which served as environmental controls. The animals were monitored and held for three weeks. No untoward effects from the vaccine were displayed and there were no signs of environmental exposure to virus.

Each vaccinate was housed so as to prevent direct dog to dog contact. This procedure was employed to prevent a vaccinate, which may be shedding, from inadvertently exposing another vaccinate which might not be immunized at the time of vaccination. The environmental controls were housed in the same area but kept physically separate from the vaccinates. These animals were bled periodically throughout the observation time and euthanized at day 21. Intestinal scraping impression slides were made and evaluated by direct fluorescent antibody staining. Serological evaluation of the serum samples drawn and evaluation of the intestinal scraping impression slides determined whether environmental exposure to a Canine Coronavirus had ocurred.

At 21 days after vaccination the vaccinates and controls were anesthetized, bled and challenged intranasally-orally with virulent challenge. The challenge virus Was titered to determine the virus input. The vaccinates, controls and challenge sentinel controls were housed in the same building. The vaccinates and controls were monitored daily for temperature elevation, lymphopenia, leukopenia and other symptoms of Corona-induced disease.

A random selection of vaccinates and controls was performed for euthanizing and evaluation of the degree of challenge virus replication in the intestinal tract on days 5, 6 and 7 following challenge. The challenge sentinel controls were euthanized and slides of intestinal tract scraping examined on days 6 and 7.

All test animals were housed in rooms where the temperature ranged from 50-70° F following challenge.

### Results

### A. Virus Input (See Table 2-1)

Ten replicate titrations were performed on the virus used to vaccinate the initial set of vaccinates. A geometric mean titer of 2.9 logs of virus was used.

### B. Comparison of Antibody Response and Overall Geometric Means

A geometric mean titer of 1:138,491 was obtained by the subcutaneous route of vaccination. The intramuscular route of inoculation elicited a geometric mean serological response of 1:113,761. No significant difference was evident between the routes of administration and the serological response obtained.

### C. Vaccine Sentinel Controls (See Table 5-1 ad 5-2)

The three dogs used as sentinel controls were shown seronegative at day 0 and at the conclusion of the study.

These three dogs were sacrificed and their intestinal tracts examined for infection by the fluorescent antibody staining of intestinal scraping impression slides. The eighteen samples examined for each animal were negative for Canine Coronavirus.

It was concluded no "wild type" Canine Coronavirus contaminated the facility to invalidate the study.

### D. Titration of the Challenge Virus

An isolate of Canine Coronavirus was obtained for intranasal oral administration to each vaccinate and challenge control dog. A geometric mean of 5.47 logs per ml. was obtained from 7 titrations. Each animal received 2 ml. of virus intranasally and 3 ml. orally. Therefore, each animal was challenged with 6.2 logs of virus.

### E. Observations and Evaluations Following Challenge

### 1. Temperature Response

One hundred thirty-one observations were made for the subcutaneously and intramuscularly vaccinated groups. No temperature response greater than 103° F was observed.

Fifty-seven temperature observations were made on the challenge control dogs. A total of 6 observations or 9.5% of the observations were over 103° F. These temperatures were 103.2, 103.2, 103.4, 103.6, 104.0 and 105.0, respectively.

The small degree of temperature response noted in the challenge control dogs was not present in the vaccinates.

### 2. White Blood Cell (WBC) Response

No significant drop in WBC was observed in either vaccinate or challenge control dogs.

### 3. Lymphocyte Response

There appeared to be a significant drop In lymphocytes for the challenge control dogs. Seven of ten challenge control dogs or 70% had lymphocyte drops of greater than or equal to 60%. Only three of the vaccinates challenged (13%) had a lymphocyte decrease of over 60% which was 5.2 fold less in the vaccinates; a 2.7 fold less drop in lymphocytes in the vaccinates as observed when considering a lymphocyte drop of 35% or greater and a 2.16 fold less drop in lymphocytes in vaccinates was observed when considering a lymphocyte drop of 25% or greater.

The data indicated that the challenge virus did have an effect on the challenge control dogs and that the vaccination prevented such a severe drop in lymphocytes in the immunized animals.

### 4. Symptoms of Disease

The symptoms of disease noted in this challenge experiment were not as prevalent as in previous experimentation. However, the challenge control dogs did demonstrate more symptoms than did the vaccinate group. A symptom of disease index was devised by dividing the number of symptoms observed per test group by the total number of observations made. For example, 3 symptoms were observed in the intramuscularly vaccinated dogs. A total of 61 observations were made. Therefore, the symptoms index was 3/61 or 0.049. Table 5-1 summarizes the symptom index per group.

**TABLE 5-1**

| Test Group | Observed Symptoms | Number of Observations | Symptom Index |
|---|---|---|---|
| Combined Vacc. | 7 | 131 | 0.053 |
| Controls | 42 | 57 | 0.737 |

A reduction in the symptom index of 92.8% was obtained when comparing the combined vaccinate group with the challenge control dogs. Although the symptoms of disease were not plentiful in the control animals, the vaccine did significantly prevent as many symptoms in the immunized animals.

Each of the ten challenge control animals demonstrated symptoms so the symptom index does not reflect a severe disease syndrome in just a few control dogs. Seven of the twenty-three vaccinates demonstrated one symptom of disease each.

### 5. Intestinal Infection - Evaluation by FA Impression Smear Procedure

The evaluation of the degree of infection of the intestinal tract by challenge virus in the control dogs and vaccinates is the most critical parameter for determining the effectiveness of a Canine Coronavirus vaccine. Intestines were removed and gently washed in cool tap water. The sections were opened with a scalpel and scrapings were taken. Scrapings were placed directly or exfoliated on glass slides from the intestinal epithelium of the test animals. Sampling occurred at appoximately the same location on each animal. The slides were fixed twice in 100% acetone at 25° C for 30 minutes. Specifically labeled Canine Coronavirus conjugate was used to stain each slide for 1 hour at 37° C. The slides were washed in a carbonate buffer wash and observed with a 50 W mercury (HBO) light source. The results are presented in Tables 5-2 through 5-6. Each field was observed and a negative to 4+ fluorescent value was given.

**TABLE 5-2**

| Observations of Intestinal Scrapings Impression Slides on Challenge Control Dogs | | | | | | | | |
|---|---|---|---|---|---|---|---|---|
| Days Post Challenge | No. of Samples | No. of Samples per Degree of FA+ | | | | | % of FA+ | Infective Index |
| | | 4+ | 3+ | 2+ | 1+ | 0 | | |
| 5 | 15 | 2 | 0 | 12 | 0 | 1 | 93.3 | 2.13 |
| 5 | 15 | 0 | 0 | 11 | 1 | 4 | 73.3 | 1.47 |
| 5 | 15 | 2 | 0 | 11 | 0 | 2 | 86.7 | 2.0 |
| 5 | 15 | 0 | 0 | 12 | 0 | 3 | 80.0 | 1.6 |
| 6 | 15 | 5 | 0 | 7 | 0 | 3 | 80.0 | 2.26 |
| 6 | 15 | 4 | 0 | 8 | 0 | 3 | 80.0 | 2.13 |
| 6 | 15 | 8 | 0 | 3 | 0 | 4 | 73.3 | 2.53 |
| 7 | 15 | 10 | 0 | 0 | 0 | 5 | 66.7 | 2.67 |
| 7 | 15 | 10 | 0 | 2 | 0 | 4 | 80.0 | 2.93 |
| 7 | 15 | 14 | 0 | 0 | 0 | 1 | 93.3 | 3.73 |
| Total | | 64 | 0 | 66 | 1 | 30 | 889.9 | 23.45 |
| Avg. | | 6.4 | | 6.6 | | 3.0 | 68.99 | 2.345 |

**TABLE 5-3**

| Observation of Intestinal Scraping Impression Slides On Dogs Euthanized 5 days After Challenge | | | | | | | | | |
|---|---|---|---|---|---|---|---|---|---|
| Vaccinate or control | Route of Vaccination | No. of Samples | No. of Samples per Degree of FA+ | | | | | % of FA+ | Infective Index |
| | | | 4+ | 3+ | 2+ | 1+ | 0 | | |
| V | SC | 15 | 0 | 0 | 0 | 2 | 13 | 13 | 0.13 |
| V | SC | 15 | 0 | 0 | 0 | 3 | 12 | 20 | 0.20 |
| V | SC | 15 | 0 | 0 | 0 | 0 | 15 | 0 | 0 |
| V | SC | 15 | 0 | 0 | 0 | 1 | 14 | 6 | 0.06 |
| V | IM | 15 | 0 | 0 | 0 | 2 | 13 | 13 | 0.13 |
| V | IM | 15 | 0 | 0 | 0 | 2 | 13 | 13 | 0.13 |
| V | IM | 15 | 0 | 0 | 0 | 0 | 15 | 0 | 0 |
| C | -- | 15 | 2 | 0 | 12 | 0 | 1 | 93.3 | 2.13 |
| C | -- | 15 | 0 | 0 | 11 | 0 | 4 | 73.3 | 1.47 |
| C | -- | 15 | 2 | 0 | 11 | 0 | 2 | 86.7 | 2.00 |
| C | -- | 15 | 0 | 0 | 12 | 0 | 3 | 80 | 1.6 |

**TABLE 5-4**

| Observation of Intestinal Scraping Impression Slides On Dogs Euthanized 6 Days After Challenge | | | | | | | | | |
|---|---|---|---|---|---|---|---|---|---|
| Vaccinate or control | Route of Vaccination | No. of Samples | No. of Samples per Degree of FA+ | | | | | % of FA+ | Infective Index |
| | | | 4+ | 3+ | 2+ | 1+ | 0 | | |
| V | SC | 15 | 0 | 0 | 0 | 1 | 14 | 6.7 | .067 |
| V | SC | 15 | 0 | 0 | 0 | 0 | 15 | 0 | 0 |
| V | SC | 15 | 0 | 0 | 0 | 5 | 10 | 33.3 | 0.33 |
| V | SC | 15 | 0 | 0 | 0 | 4 | 11 | 26.7 | 0.267 |
| V | IM | 15 | 0 | 0 | 0 | 0 | 15 | 0 | 0 |
| V | IM | 15 | 0 | 0 | 0 | 0 | 15 | 0 | 0 |
| V | IM | 15 | 0 | 0 | 0 | 0 | 15 | 0 | 0 |
| V | IM | 15 | 0 | 0 | 0 | 0 | 15 | 0 | 0 |
| C | -- | 15 | 5 | 0 | 7 | 0 | 3 | 80.0 | 2.27 |
| C | -- | 15 | 4 | 0 | 8 | 0 | 3 | 80.0 | 2.13 |
| C | -- | 15 | 8 | 0 | 3 | 0 | 4 | 73.3 | 2.53 |
| Sentinel Control | -- | 15 | 0 | 0 | 0 | 0 | 15 | 0 | 0 |

**TABLE 5-5**

| Observation of Intestinal Scraping Impression Slides On Dogs Euthanized 7 Days After Challenge | | | | | | | | | |
|---|---|---|---|---|---|---|---|---|---|
| Vaccinate or Control | Route of Vaccination | No. of Samples | No. of Samples per Degree of FA+ | | | | | % of FA+ | Infective Index |
| | | | 4+ | 3+ | 2+ | 1+ | 0 | | |
| V | SC | 15 | 0 | 0 | 0 | 1 | 14 | 6.67 | .067 |
| V | IM | 15 | 0 | 0 | 0 | 8 | 7 | 53.3 | .533 |
| V | IM | 15 | 0 | 0 | 0 | 2 | 13 | 13.3 | .133 |
| V | IM | 15 | 0 | 0 | 0 | 3 | 12 | 20.0 | .2 |
| V | IM | 15 | 0 | 0 | 0 | 0 | 15 | 0 | 0 |
| V | IM | 15 | 0 | 0 | 0 | 0 | 15 | 0 | 0 |
| V | IM | 15 | 0 | 0 | 0 | 0 | 2 | 13 | .133 |
| C | -- | 15 | 10 | 0 | 0 | 0 | 5 | 66.7 | 2.67 |
| C | -- | 15 | 10 | 0 | 0 | 2 | 3 | 80 | 2.93 |
| C | -- | 15 | 14 | 0 | 0 | 0 | 1 | 93.3 | 3.73 |
| Sentinel Control | -- | 15 | 0 | 0 | 0 | 0 | 15 | 0 | 0 |

**TABLE 5-6**

| Infective Index Comparison Indicating Reduction in Intestinal Infection | | | |
|---|---|---|---|
| Test Group | Infective Index per Day of Euthanizing | | |
| | 5 | 6 | 7 |
| SC Vaccinates | 0.0975 | 0.132 | 0.067 |
| IM Vaccinates | 0.13 | 0 | 0.217 |
| Combined Groups | 0.108 | 0.066 | 0.187 |
| Challenge Controls | 1.8 | 2.31 | 3.11 |
| Percent Reduction Combined Group vs Controls | 94% | 97% | 94% |

The challenge control infective index ranged from 1.47 to 3.73 with an average of 2.345, where as the combined vaccinate group of 22 dogs had an infective index of 0.103. These data indicate that a reduction of intestinal infection of 95.6% was achieved in the vaccinates.

### EXAMPLE 6

### This example illustrates Canine Coronavirus as a combination product.

A pilot serial of vaccine was batched with Canine Coronavirus and Canine Parvovirus as components as follows:

| | |
|---|---|
| CCV fluids | 4.5 liters |
| CPV fluids | 2.0 liters |
| Stabilizer | 2.2 liters |
| Fetal Calf Serum | 0.1 liters |
| 1 N NaOH | 0.08 liters |

The materials were blended under refrigeration and bottled aseptically in a 1 ml. dose. The bottles of vaccine were then subjected to desiccation. The virus yields were as follows:

| | |
|---|---|
| CCV | 10^{5.4}FAID₅₀/dose |
| CPV | 10^{6.5}FAID₅₀/dose |

It is quite feasible to routinely produce and batch CCV as a combination product.

This portion of the study was designed to determine if antigen interference would occur when Canine Coronavirus and Canine Parvovirus were combined for administration as a combination vaccine.

The experimental design can be found in Table 6-1. Three groups of dogs were used. Group I was vaccinated with a full field dose of Canine Coronavirus vaccine (1 ml.). Group II was vaccinated with a full field dose of Canine Corona-Parvovirus vaccine (1 ml.). The third group was vaccinated with a full field dose of Canine Parvovirus (1 ml.). Portions of each group were vaccinated intramuscularly (IM) while the remaining dogs were vaccinated subcutaneously (SC). The dogs were bled on day 0 and also 21 days after vaccination. Sentinel controls were also bled on day 0 and day 21.

**TABLE 6-1**

| Antigen Blockage Study Experimental Design | | | |
|---|---|---|---|
| Treatment | Group I | Group II | Group III |
| Prevaccination Bleeding and Vaccination (day 0) | 4 vaccinated | SC 3 vaccinated | SQ 3 vaccinated SQ |
| | 4 vaccinated | IM 5 vaccinated | IM 4 vaccinated IM |
| Post vaccination bleeding (day 21) | bled | bled | bled |

Five replicate titrations were performed on each virus fraction. The data indicated a virus input of 4.5 logs of Coronavirus for Group I, 5.5 logs of Canine Parvovirus for Group II, and 4.4 logs of Canine Coronavirus with 5.5 logs of Canine Parvovirus for Group II.

### 1. Test Group I - Canine Coronavirus Vaccinate

The data indicated that a geometric mean titer of 1:6498 is obtained against Canine Coronavirus.

### 2. Test Group II-Canine Corona-Parvovirus Vaccinate

The dogs in test Group II responded to a geometric mean titer of 1:87,222 against Canine Coronavirus and a geometric mean titer of 1:58,579 against Canine Parvovirus 21 days after receiving 1 field dose of vaccine.

### 3. Test Group III-Canine Parvovirus Vaccinate

A geometric mean titer of 1:65,893 was obtained in this test group against Canine Parvovirus.

The results are summarized in Table 6-2.

**TABLE 6-2**

| Post Serological Canine Corona and Parvo Response Arithmetic and Geometric Means Summary | | | | | |
|---|---|---|---|---|---|
| Group | Vaccine | Post Vaccination Antibody Titer | | | |
| | | AM* | | GM** | |
| | | CCV | CPV | CCV | CPV |
| I | CCV | 64,111 | --- | 6,498 | --- |
| II | CCV-CPV | 163,436 | 128,172 | 87,222 | 58,579 |
| III | CPV | --- | 391,982 | --- | 65,893 |

| | | | | | |
|---|---|---|---|---|---|
| * Arithmetic Mean | | | | | |
| ** Geometric Mean | | | | | |

### Analysis for Antigen Blockage

### a. Canine Coronavirus (See Table 6-2)

No antigen blockage was noted for the Canine Coronavirus. A geometric mean antibody titer of 1:87,222 was achieved with the combination product as compared to a geometric mean antibody level of 1:6498 with the monovalent product against Canine Coronavirus. A potentiation effect is achieved with the Canine Coronavirus in combination. A 13.4 fold increase in Canine Coronavirus antibody titer is realized when comparing the combination product to the monovalent product.

### b. Canine Parvovirus

There was no significant difference in antibody titers against Canine Parvovirus when comparing the combination product with the monovalent product. A geometric mean titer of 1:58,579 with the combination product compares well with a geometric mean titer of 1:65,893 with the monovalent product. No antigen blockage was observed.

### EXAMPLE 7

### This example illustrates an inactivation procedure.

Non-frozen canine coronavirus fluids produced as described in Example 1 were inactivated by binary ethylenimine inactivation (BEI). Stock Solution A of 0.2 molar bromoethylamine hydrobromide was prepared by adding 40.98 grams to deionized water and made up to 1000 ml. Solution B was sodium hydroxide, 0.4 molar. This was prepared by adding 16 grams of NaOH to deionized water and made up to 1000 ml. The stock solutions were stored at room temperature until ready for use. Prior to usage equal volumes of Solution A and B were mixed and incubated at 37° C to cyclize. The cyclized solution was then added at 2 percent vol/vol concentration to the CCV fluids. The fluids were mixed thoroughly and incubated at 37° C for 72 hours. At the end of this incubation 20 ml. of a sterile 1 molar sodium thiosulfate solution was added to insure neutralization of the BEI. Diluted and undiluted Samples of the inactivated fluids were placed in susceptible tissue culture to detect any non-inactivated virus. The tissue culture was passaged three times and examined via fluorescent antibody using specific CCV conjugate. Tests indicated complete inactivation.

### EXAMPLE 8

### This example illustrates an alternate inactivation procedure.

Non-frozen canine coronavirus fluids produced as described in Example 1 were inactivated with beta-propriolactone (BPL). The virus fluids at a 1 liter volume were buffered with 5 ml. of 1 normal sodium hydroxide to compensate for the pH change resulting from the acidic products released from the beta-propriolactone hydrolization. One half of one ml. of concentrated BPL was added to the fluids. The fluids were held at 4° C± 1° C for 24 hours with periodic agitation. After which time the fluids were sampled, passaged on tissue culture, and tested via fluorescent antibody testing for any non-inactivated virus. Tests indicated complete inactivation.

### EXAMPLE 9

### This example illustrates adjuvanting the inactivated virus.

Adjuvant 9A, ethylene maleic anhydride, was prepared in the following manner. EMA 31 at 150 grams of Monsanto lot LC07 was dissolved in 3 liters of deionized water and heated to 85°C. The mixture was agitated until the EMA 31 was visibly dissolved. Phenol red, lot number 7715, ICN Pharmaceuticals, was added at 0.03 grams. The pH of the preparation was adjusted to 6.8 with 175 milliliters of 10 normal sodium hydroxide, Eastman Kodak Co. The adjuvant was dispensed into 6 glass screw cap bottles at 500 milliliters each and subjected steam sterilization at 15 pounds per square inch pressure and a temperature of 121 degrees centigrade for 2 hours.

Adjuvant 9B, a CSMA aqueous suspension, was prepared in the following manner. Neocryl A-640 lot RL-414 at 7.5 liters was mixed with 7.5 liters of deionized water containing 70 grams of sodium chloride, lot KMPA, Mallinckrodt. The preparation was mixed and dispensed into 30-500 milliliter glass screw top containers. These were subjected to steam sterilization at 15 pounds per square inch pressure and 121 degrees centrigrade temperatures for 2 hours.

Killed canine coronavirus antigen at 25 liter was combined with 271.7 milliliters of adjuvant 9A. There was a pH shift to acid and this was adjusted with 21 milliliters of 1 normal sodium hydroxide resulting in a pH of 7.5. To the mixture 1902.6 milliliters of adjuvant 4B was added. The entire bulk vaccine was mixed and filled into 18,850 13 millimeter finish type I minivial vaccine containers for testing and evaluation.

### EXAMPLE 10

### This example illustrates alternative adjuvanting of the inactivated virus.

One liter of canine coronavirus fluids which were inactivated with 2 percent BEI was used in this process. The adjuvants were prepared in the following manner:
ADJUVANT 10A - Ethylene-maleic anydride (EMA) solution was prepared in the following manner. Phosphate buffered saline (PBS) was prepared at 0.01 M. in deionized water. Eight grams of sodium chloride (NaCl) per liter was added with 0.2 grams of potassium chloride (KCl), 1.15 grams of sodium phosphate dibasic (Na₂HPO₄) and 0.2 grams of potassium phosphate mono basic (KH₂PO). Sixty three hundred ml. of PBS were used in preparing the EMA. The PBS was heated to 100° C. Three hundred and fifteen grams of EMA 31 #LCO7329 (Monsanto Co, 800 N. Lindberg Blvd., St. Louis, Missouri 63166) was added and dissolved in the PBS. Phenol red at 0.05 grams was added to facilitate gross pH adjustments. Once dissolved the pH of the solution was adjusted to 6.8 with 260 ml. of 10 normal sodium hydroxide (NaOH). The solution was dispensed into 500 ml. screw cap bottles and sterilized by autoclaving at 121° C at 15psi for 2 hours.
ADJUVANT 10B - CSMA suspension diluted in PBS was prepared in the following manner. Five gallons of the same PBS as described above with regard to adjuvant 10A was mixed with 5 gallons of Neocryl A640 lot RC-4570 (Polyvinyl Chemical Industries, 730 Main Street, Wilmington, Mass. 01887). The suspension was thoroughly mixed and dispensed into 500 ml. screw top bottles and 12 liter jugs. These vessels were autoclaved for 3 hours at 121° C, 15psi to sterilize.

One liter of the CCV inactivated fluids were mixed thoroughly via a magnetic mixer. Sixty ml. of adjuvant 10A was added. Immediately, 10 ml. of 1 normal NaOH was added to adjust the pH to 7.34 (as determined by an Orion pH meter). One hundred ml. of adjuvant 10B was added. The mixture was allowed to mix for 10 minutes then dispensed into aliquots for animal testing.

### EXAMPLE 11

### This example illustrates the effectiveness of various adjuvants with inactivated canine coronaviruses (CCV) KV.

Fifty-two dogs seronegative and susceptible to CCV were used to demonstrate the effectiveness of several adjuvant systems with a killed canine coronavirus vaccine. All test dogs were parenterally inoculated with one dose of product. A two dose vaccination regimen was used. Serum neutralizing antibody levels were determined 3 weeks post one inoculation and two weeks post a second inoculation.

| Reciprocal Geometric Mean Serum Neutralizing Antibody Levels | | | | | |
|---|---|---|---|---|---|
| Number of Test Dogs | Antigen Level | Adjuvant types used | Pre Vaccination | 3 wks. post 1st 1st vaccination | 2 wks post 2nd vaccination |
| 11 | Minimum | EMA 31 & Neocryl A640 | < 2 | 8.9 | 48 |
| 12 | Minimum | EMA 31 & Neocryl A640 | < 2 | 17.8 | 30.7 |
| 5 | Full | EMA 31 & Neocryl A640 | < 2 | 19 | 62 |
| 4 | Full | Neocryl A640 | < 2 | 16 | 28 |
| 5 | Full | Aluminum Phosphate | < 2 | 17.5 | 32 |
| 5 | Full | Aluminum Hydroxide Alhydrogel | < 2 | 12.5 | 38 |
| 5 | Full | Aluminum Hydroxide Reheis | < 2 | 38.2 | 57.6 |
| 5 | Full | Neocryl & Aluminum Phosphate | < 2 | ≦ 9.9 | 53.9 |
| Alhydrogel and Reheis are trade names for aluminum hydroxide. | | | | | |

The data indicates that the combined adjuvant system of EMA-31 and Neocryl-A640 is superior.

### EXAMPLE 12

### This example illustrates the procedure for vaccine evaluation.

This study was designed to determine the feasibility and degree of efficacy of an inactivated coronavirus vaccine. The adjuvanted product described in Example 10 was utilized for these studies. The study would also determine whether one dose or two would be required to adequately protect the canine. Ten dogs, which were tested by serum neutralization tests to be CCV seronegative, were utilized in the two-part study. Six of the dogs, numbers 214, 222, 219, 220, 212 and 213, were given a one ml. dose of vaccine administered intramuscularly. These dogs were bled on days 4, 7, 11, 14 and 21 post first vaccination to serologically monitor the response to the vaccine. Dogs 214 and 222 along with challenge controls, 244 and 245, were challenged intranasalIy-orally with I-171 virus (ATCC VR-809). At 5 days post challenge the four animals were euthanized and examined for evidence of coronaviral infection. Gross pathology showed a remarkable inflammation of the small intestines in the challenge controls (244 and 245). The vaccinates however, showed slight, if any, signs of inflammation. The same held true on examination of the pancreas. Samples of the brain, meninges, lungs, liver, spleen, mesentary, mesentary lymph nodes, pancreas and 10 samples at equal distance and locations in the intestines were taken from each dog for CCV detection by exfoliation fluorescent antibody. Results are presented in Table 12-1.

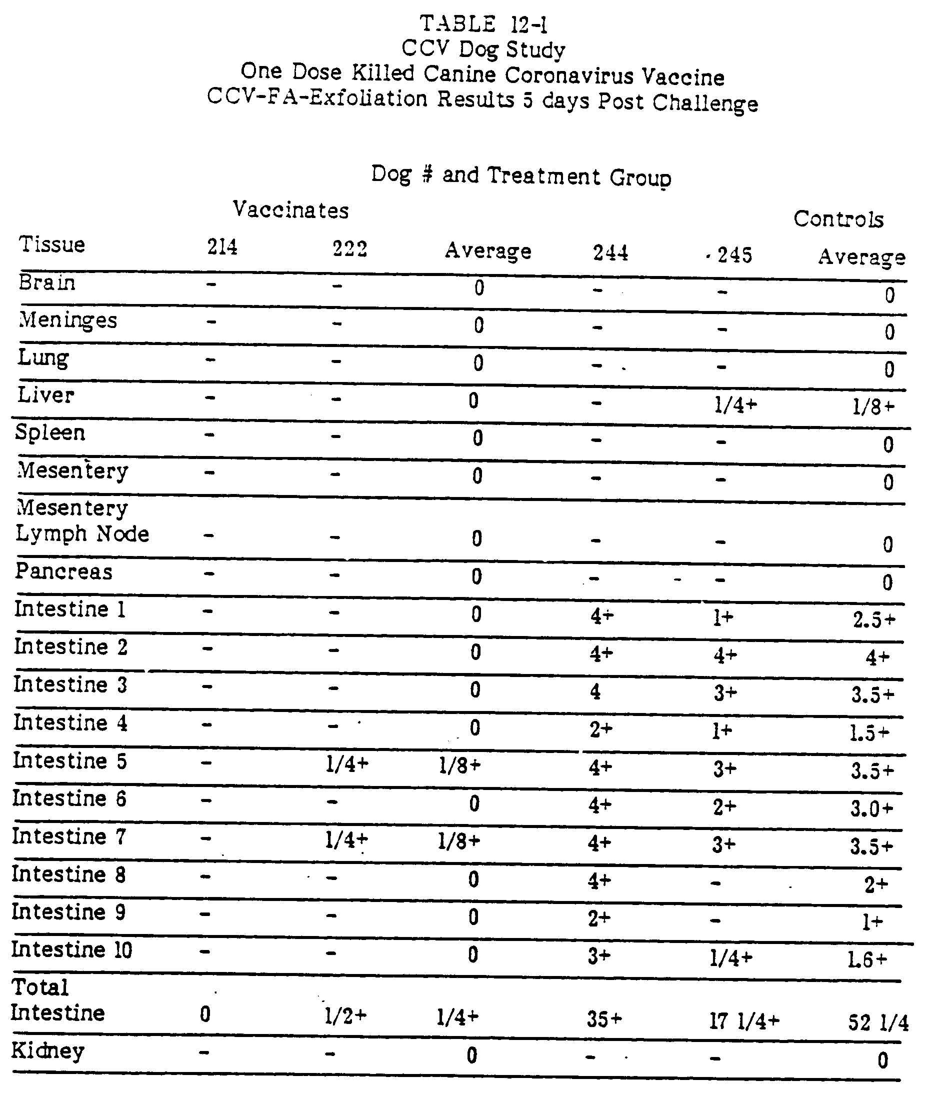

In comparing the degree of infection as evidenced by the exfoliation fluorescent antibody it was apparent that the vaccine was efficacious in reducing viral infection of the intestines. An overall fluorescent antibody (FA) average of 52 1/4+ was demonstrated in the challenge controls. The vaccinates were demonstrated to have a 1/4 + FA average. This indicated a 99.5 percent reduction in the vaccinates as compared to challenge controls. A modified live vaccine had provided a 95 percent reduction therefore, establishing that the protection afforded by the inactivated vaccine were comparable.

Serum neutralization (SN) tests on the 14 and 21 post vaccination and 5 day post challenge bleedings were conducted. "KV" denotes "killed virus." Results are presented in Table 12-2.

**TABLE 12-2**

| SN Results Post One Dose of KV CCV and Comparison with Challenge Controls | | | | |
|---|---|---|---|---|
| Animal Designation | Vaccine | SN 14 days post vaccination | 21 days post vaccination & day of challenge | 5 days post challenge |
| 214 | 1 dose KV CCV | 1:224 | 1:224 | 1:851 |
| 222 | 1 dose KV CCV | 1:34 | 1:59 | 1:224 |
| 244 | None:Challenge Control | Not Tested | < 1:2 | 1:5 |
| 245 | None:Challenge Control | Not Tested | < 1:2 | 1:5 |

From Table 12-2, it is apparent that a 1:59 SN antibody level is sufficient for protection. Both vaccinates responded with one dose of vaccine to have a sufficient serum neutralization antibody titer along with local target immunity to provide protection against infection. The challenge controls at the time of challenge serologically responded further proving that they were challenged. The vaccinates at 5 days post challenge responded at the first stages of an amnestic response.

The second dose phase of the study began 21 days post first vaccination. The four remaining dogs were given a second 1 ml. dose of the inactivated CCV vaccine, administered intramuscularly.

At two weeks post second vaccination the four vaccinates, 212, 213, 219 and 220 were challenged along with 2 susceptible corona control dogs, 270 and 271. The I-171 (ATCC VR-809) coronavirus was used as challenge. Five ml. per dog was administered intranasally and orally. At five days post challenge, all six dogs were euthanized and samples of the intestines, spleen, brain and meninges were taken for exfoliation fluorescent antibody testing for canine coronavirus detection. Ten samples of the intestines were taken with only individual samples of the other organs being taken. Exfoliation results are summarized in Table 12-3.

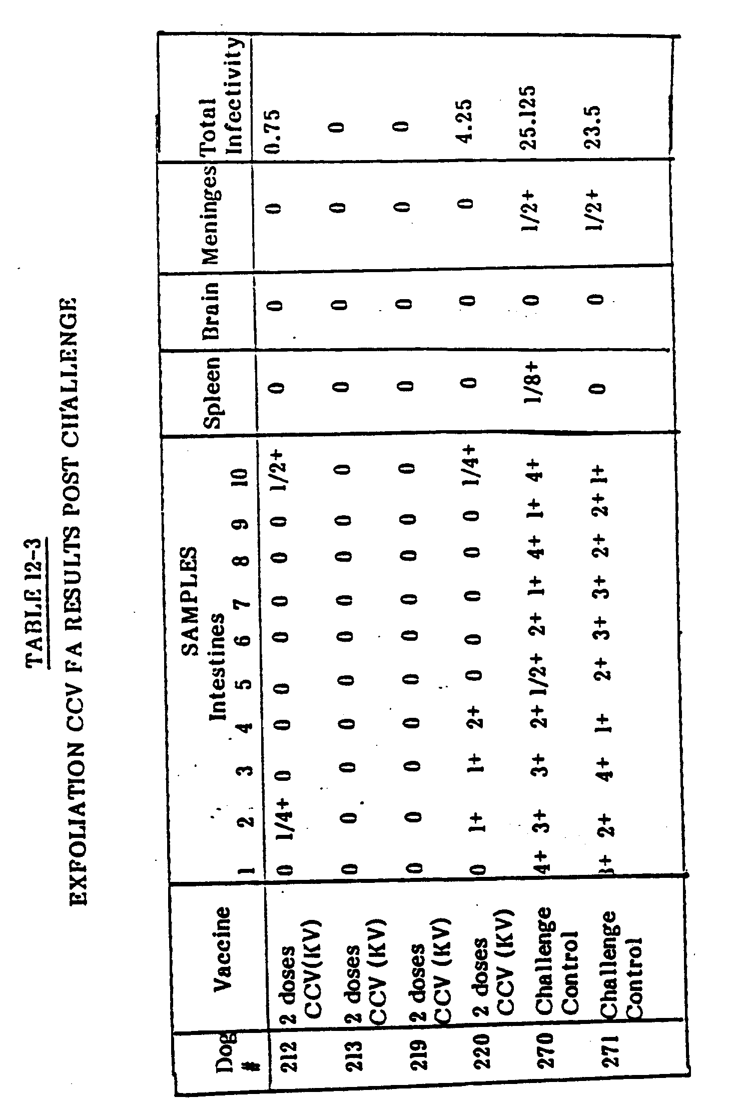

The average for the two controls for CCV infectivity was 24.3. The average for the four vaccinates was 1.25. The overall reduction in infectivity was 94.9 percent in comparison of vaccinates to controls. It was noted that CCV viral replication in the meninges occurred in the controls. No indication of virus was seen in the meninges of the vaccinated animals after challenge. Protection against meningial injection was therefore afforded the vaccinates.

### EXAMPLE 13

### This example demonstrates the compatibility of CCV KV with other virus fractions.

The objective of this study was to demonstrate that the killed Canine Coronavirus vaccine can be used in combination with Canine Distemper virus, Canine Adenovirus Type 2, Canine Parainfluenza and Canine Parvovirus vaccines.

### Experimental Design

1. Vaccination
   Twenty-one seronegative dogs were vaccinated with a 1 ml. volume of the immunogenicity serial. Ten dogs were subcutaneously vaccinated and eleven dogs were intramuscularly vaccinated.
   The twenty-one vaccinates and seven environmental controls were bled at the time of vaccination, at 21 days post vaccination at which time the vaccinates were reinoculated, and at 14 days post second vaccination.
   Antibody level determinations were made on all vaccinates for all five viral agents to demonstrate the level of antibody response which can be expected with the combination product [DA₂PC(KV)-CPV(MLV)]. The purpose of this data was to determine if antigen blockage occurs.

### Antigen Compatibility (See Table 13-1)

Table 13-1 is a summary table citing the geometric mean antibody titers for each of the five fractions contained in the combination product.

The data establishes the antibody titers expected with a minimum potency KV combination serial. The data indicates all vaccinates responded to vaccination and that no antigen blockage occurred. The challenge data establishes the immunological properties of the KV CCV fraction were not interfered with by the four other virus fractions (CDV, CAV-2, CPI, CPV) which were present in the vaccine.

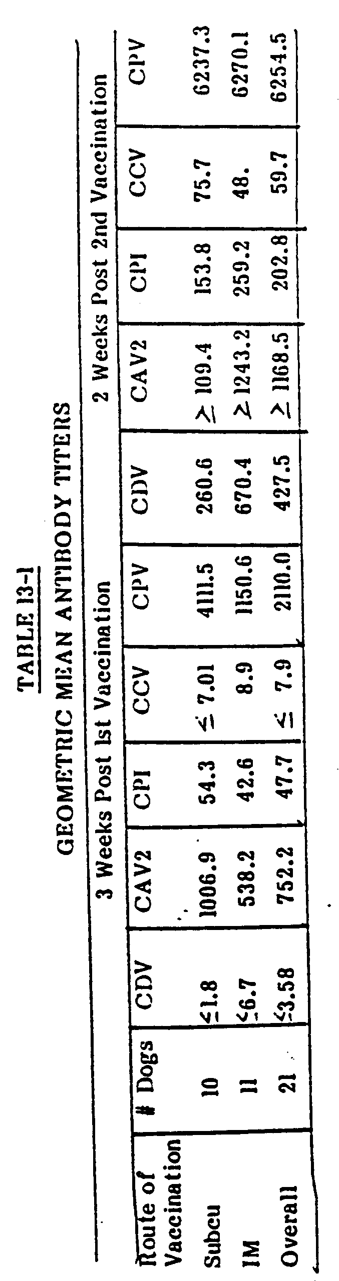

### EXAMPLE 14

### This example illustrates canine coronavirus cross neutralization between five various isolates.

Studies were conducted to determine the cross neutralization relationships (serum neutralization of virus) between various isolates of canine coronavirus. Specific antiserum to each individual isolate was prepared in susceptible dogs. Serum neutralization (SN) tests with the various isolates at constant levels or titers were run against twofold dilutions of the specific antisera. In other words the antibody induced in the dogs by a specific isolate was evaluated as to the antibody's ability to neutralize various other virus isolates as well as the identical virus isolate used to induce the antibody. A virus isolate which induced antibody which will neutralize the most virus isolates is the best vaccine virus candidate. Results of those tests are depicted in the following table. The specific antisera are presented vertically against the various isolates listed horizontally. The reciprocal SN level is expressed.

| Antisera produced by indicated CCV | Antibody level against the virus inducing antibody formation | Antibody levels against other viruses | | | | |
|---|---|---|---|---|---|---|
| | | I-171 | K-378 | S-378 | A76-5 | ATCC VR 2068 |
| I-171 | 89 | - | 128 | 89 | 20 | 45 |
| K-378 | 45 | 22 | - | 89 | 3 | 40 |
| S-378 | 178 | 178 | 148 | - | 6 | 37 |
| A76-5 | 11 | 45 | 32 | 64 | - | 50 |
| ATCC No. VR 2068 | 45 | 64 | 128 | 54 | 25 | - |

The data was analyzed from the standpoint of the antibody which would neutralize all five viruses. The antibody induced by the ATCC No. VR2068 virus isolate neutralized the other four viruses to a significant degree.

The A76-5 antiserum and virus proved to be the least cross reactive of the five isolates tested. The ATCC VR 2068 proved to have a significant level of cross reactivity with this virus isolate.

The data also indicates that there are no major serological differences among these five canine coronaviruses. The I-171 and ATCC VR 2068 viruses are indicated as the best choices for vaccine strains; however due to the cross neutralization between all the isolates any of the canine coronaviruses could be used as an antigen.

### EXAMPLE 15

### This example illustrates Vaccine Efficacy.

Twenty-eight dogs seronegative to CDV, CAV₂, CPI, CCV and CPV were used as vaccinates and sentinel controls. Twenty-one dogs were used as vaccinates and seven dogs were used as environmental or sentinel controls. The seven environmental controls along with three additional dogs seronegative to CCV were used as challenge control dogs. Thirty-one dogs were used for the entire study.

Twenty-one seronegative dogs were vaccinated with a 1 ml. volume of the immunogencity serial. Ten dogs were subcutaneously vaccinated and eleven dogs were intramuscularly vaccinated. The twenty-one vaccinates and seven environmental controls were bled at the time of vaccination, at 21 days post vaccination at which time the vaccinates were reinoculated, at 14 days post second vaccination and at 28 days post second vaccination which was also the day of challenge.

Antibody level determinations were made on all environmental control for all 5 viral agents to demonstrate that no exposure to extraneous virus occurred during the course of the study. Pre-challenge antibody level determinations were made to demonstrate the susceptibility of the challenge control dogs to CCV.

The 21 vaccinates and 10 control dogs were bled, anesthetized and challenged with the CCV I-171 challenge virus identity. Each dog received 2 ml. per naris and 3 ml. orally or 5 ml. of challenge per dog. White blood cell-lymphocyte counts were performed 2 days prior to challenge and 6 to 7 days following challenge.

Five dogs vaccinated by the subcutaneous route, five dogs vaccinated by the intramuscular route and five challenge control dogs were euthanized 6 days following challenge. On day 7 post challenge five dogs vaccinated by the subcutaneous route, six dogs vaccinated by the intramuscular route and five challenge controls dogs were euthanized.

The small intestine was removed and 10-13 inch sections from each dog was used for sampling. The ten sections were taken from the same areas in each dog. The first section (sample 1) was taken just below the stomach and the last section (sample 10) was obtained just above the small intestine-large intestine junction. Each section was scraped and the intestinal samples suspended in PBS. The tubes containing the cell suspension were grossly observed for appearance. Cell samples from each tube were placed on slides which were air dried, acetone fixed and stained with canine coronavirus antibody conjugate. These slides were then evaluated to determine the degree of viral infection which existed in the cells.

### RESULTS AND DISCUSSION

### A. White Blood Cell Counts Post Challenge

A white blood cell count reduction of greater than 45% from the baseline was used for purposes of evaluating this study.

### Vaccinates

Six observations of WBC drops greater than 45% from baseline were recorded out of the 65 WBC counts performed on the dogs inoculated subcutaneously. The WBC count index number (6 divided by 65) was 0.092.

Three observations of WBC drops greater than 45% from baseline were recorded out of the 72 WBC counts performed on the dogs inoculated intramuscularly. The WBC count index number (3 divided by 72) was 0.042.

### Controls

Twenty observations of WBC drops greater than 45% from baseline were recorded out of the 65 WBC counts performed on the challenge control dogs. The WBC count index number (20 divided by 65) was 0.308 for the challenge controls. Another way of expressing this data is that 30.8% of the WBC counts performed on the challenge control dogs were 45% below the pre-challenge baseline counts.

### Discussion

The percent prevention of WBC count decreases below 45% when comparing vaccinates to controls is 79.6%. The majority of the WBC decreases occurred on days 2 and 3. Table 15-1 compares the WBC drops of vaccinates versus controls on a daily basis.

**Table 15-1**

| Days Post Challenge | Percentage of dogs showing a WBC drop of 45% or greater below baseline | |
|---|---|---|
| | Controls | Vaccinates |
| 1 | 0 | 0 |
| 2 | 20 | 28.6 |
| 3 | 70 | 9.5 |
| 4 | 50 | 0 |
| 5 | 20 | 0 |
| 6 | 20 | 4.8 |
| 7 | 40 | 0 |

The vaccine therefore provided significant protection to the vaccinates in the prevention of drops in WBC counts

### B. Lymphocyte Cell Count Drops Post Challenge

A lymphocyte cell count reduction of greater than 50% from the baseline was used for purposes of evaluating this study.

### Vaccinates

Six observations of lymphocyte drops greater than 50% from baseline were recorded out of 65 lymphocyte counts performed on the challenge control dogs. The lymphocyte count index number (16 divided by 65) was 0.246 for the control dogs.

Three observations of lymphocyte drops greater than 50% from baseline were recorded out of the 72 lymphocyte counts performed on the dogs inoculated intramuscularly. The lymphocyte count index number (3 divided by 72) was 0.042 for this group.

### Controls

Sixteen observations of lymphocyte drops greater than 50% from baseline were recorded out of 65 lymphocyte counts performed on the challenge control dogs. The lymphocyte count index number (16 divided by 65) was 0.246 for the control dogs.

The lymphocyte drops occurred most frequently on day 4 post challenge with 7 of 10 dogs (70%) demonstrating a drop in lymphocytes. Two other days showing a high frequency of lymphocyte drops post challenge were day 3 with 5 of 10 dogs (50%) and day 2 with 3 of 10 (30%).

### Discussion

The percent prevention of lymphocyte count decreases below 50% when comparing vaccinates to controls was 73.2%.

The majority of lymphocyte decreases occurred on days 2, 3 and 4 post challenge. Table 15-2 compares the lymphocyte drops of vaccinates versus controls on days 2, 3 and 4 post challenge.

**Table 15-2**

| Days Post Challenge | Percentage of dogs showing lymphocyte drop of 50% or greater below baseline | |
|---|---|---|
| | Controls | Vaccinates |
| 2 | 9.5 | 30.0 |
| 3 | 19.0 | 50.0 |
| 4 | 4.8 | 70.0 |

The data demonstrates that the killed CCV vaccine provided significant protection to the vaccinates in the prevention of drops in lymphocytes following challenge.

### C. Intestinal Infection with Canine Coronavirus Post Challenge Vaccinates

The 210 intestinal samples were examined for the presence of CCV virus by the fluorescent antibody staining technique. Five of the 210 samples (2.4%) were positive for virus. The degree of infection did not exceed an 1/8+.

No fluorescence typical of CCV virus was observed in the meninges of any of the vaccinates.

The number of samples positve per degree of infection (4+ - 1/8+) were totaled and the Total FA + for each dog determined. The infective index for each dog was calculated by the following method.

A group infective index was obtained by totaling the individual dogs infective index and dividing by the total number of dogs in each group.

The infective index for the subcutaneously vaccinated dogs was 0.0025 while the infective index for the intramuscularly vaccinated dogs was 0.0034.

The infective index for the vaccinates examined on day 6 versus those examined on day 7 did not vary significantly.

### Controls

The 100 intestinal samples were examined for the presence of CCV virus by the fluorescent antibody staining technique. Eighty-one (81) of the 100 samples (81%) were positive for virus. The degree of infection ranged from a 4+ (highly infected samples) to negative. All ten challenge control dogs (100%) demonstrated CCV infection of the intestinal tract.

Three of the 10 challenge control dogs (30%) demonstrated fIuorescence typical of CCV in their meninges.

The number of samples positive per degree of infection (4+ to 1/8+) were tallied. The same calculation methods used above were used for this data.

The infective index for the challenge control dogs is 1.594. The infective index for the challenge control dogs examined on day 6 versus those examined on day 7 did vary to some degree. Ninety percent of the samples of the control dogs examined on day 6 were positive while seventy-two percent of the samples of the control dogs examined on day 7 were positive. The degree of infection was 36.5% less on day 7 versus day 6. All of the control dogs were infected with CCV.

### Discussion

A comparison was made of vaccinates and controls in relation to the severity and incidence of intestinal infection. The data was also broken down between the 6 day and 7 day post observation periods. The results indicate the degree of protection provided is not dependent upon the route of vaccination.

The vaccine reduced the degree of infection in the vaccinates by at least 99.7%. The data based on the incidence of positive samples per test group irregardless of the degree of infection indicates the vaccine reduced the degree of infection in the vaccinates by at least 95.6%. The data obtained from the samples observed on day 6 and 7 post challenge shows 98.1% reduction in intestinal infection as measured by severity and incidence was provided by the vaccine. A 97% reduction in intestinal infection as measured by incidence was provided by the vaccine. The test parameters of temperature response, white blood cell response and lymphocyte response for both vaccinates and controls post challenge were summarized. It is pointed out that CCV virus was observed in the meninges of 30% of the controls whereas no CCV virus was observed in any of the vaccinates meninges.

### D. Serological Data

### Environmental Controls

Antibody level determinations on the seven environmental controls indicated no exposure to extraneous CDV, CAV-2, CPI or CPV virus occurred during the course of the experiment. These antibody level tests were conducted on blood specimens drawn five weeks post 1st vaccination which also represents the 2 weeks post second vaccination time period.

### Pre and Post Vaccination Antibody Levels to Canine Coronavirus

### Vaccinates

The antibody titers of the vaccinates at the time of vaccination, at 3 weeks post 1st vaccination, 2 weeks post second vaccination, 4 weeks post second vaccination and 6 or 7 days post challenge indicate all vaccinates were seronegative prior to vaccination. The data indicates 20 of 21 vaccinates (95.2%) serologically responded to the first vaccination. The geometric mean titer was 1:7.9. The two weeks post second vaccination geometric mean titer increased 7.6 fold to a 1:59.7 titer. All vaccinates had SN titers of greater than or equal to a 1:9 SN titer. The four week post second vaccination geometric mean titer increased another 1.57 fold to a 1:93.9 titer. The lowest antibody titer recorded at the 4 week post second vaccination period was 1:34. The subcutaneous route of vaccination appeared to elicit a somewhat higher antibody titer than did the intramuscular route of vaccination.

The post challenge SN antibody levels indicate a lower antibody level was observed in the vaccinates following challenge. The 5 and 6 day post challenge bleeding time is probably too early for amnesis to occur. It may be that the challenge virus-antibody interaction could account for the lower SN responses observed.

### Controls

Seven of the ten control dogs served as environmental controls as well as challenge control dogs. The dogs were seronegative at the time of challenge thus proving no environmental exposure to live CCV virus occurred during the study. This also proved their susceptibility to the challenge virus.

The 6 and 7 day post challenge antibody levels remained low. This is not unexpected. A measurable antibody response to parenteral CCV inoculation is 6-9 days. The FA observations of intestinal tract samples indicate the virus was present in the dogs but seroconversion had not been accomplished in a 7 day interval between challenge and bleeding prior to euthanizing of the dogs.

The foregoing results demonstrates the satisfactory immunogenicity of a killed Canine Coronavirus (CCV) vaccine.

## Claims (Claims for the following Contracting State(s): BE, CH, DE, FR, GB, LI, LU, NL, SE)

1. A veterinary vaccine composition comprising viral antigen, a pharmaceutically acceptable carrier, and an adjuvant which is at least one member selected from ethylene maleic anhydride and a copolymer of styrene with a mixture of acrylic acid and methacrylic acid.

2. A composition as claimed in claim 1 in which the viral antigen is that of canine coronavirus.

3. A composition as claimed in claim 2 in which the viral antigen is the avirulent antigenic product produced by either
(a) attenuating live canine coronavirus by passage through cells of feline origin such that when administered to a dog by injection the attenuated live virus selectively infects the intestinal epithelium, or
(b) inactivating feline or canine cell propagated canine coronavirus.

4. A composition as claimed in claim 3 wherein the avirulent product is an inactivated canine coronavirus product having a pre-inactivation titer of about 10,000 to 10,000,000 virus particles per dose as measured by the FAID₅₀ method.

5. A composition as claimed in any one of claims 1 to 4 wherein the adjuvant is ethylene maleic anhydride.

## Claims (Claims for the following Contracting State(s): AT)

1. A process for preparing a veterinary vaccine composition which comprises bringing into association a viral antigen, a pharmaceutically acceptable carrier and an adjuvant which is at least one member selected from ethylene maleic anhydride and a copolymer of styrene with a mixture of acrylic acid and methacrylic acid.

2. A process as claimed in claim 1 in which the viral antigen is that of canine coronavirus.

3. A process as claimed in claim 2 in which the viral antigen is the avirulent antigenic product produced by either
(a) attenuating live canine coronavirus by passage through cells of feline origin such that when administered to a dog by injection the attenuated live virus selectively infects the intestinal epithelium, or
(b) inactivating feline or canine cell propagated canine coronavirus.

4. A process as claimed in claim 3 wherein the avirulent product is an inactivated canine coronavirus product having a pre-inactivation titer of about 10,000 to 10,000,000 virus particles per dose as measured by the FAID₅₀ method.

5. A process as claimed in any one of claims 1 to 4 wherein the adjuvant is ethylene maleic anhydride.

## Patentansprüche (Patentansprüche für folgende(n) Vertragsstaat(en): BE, CH, DE, FR, GB, LI, LU, NL, SE)

1. Veterinärvakzinezusammensetzung, umfassend ein Virusantigen, einen pharmazeutisch akzeptablen Träger und ein Adjuvans, u.zw. mindestens ein Mitglied, ausgewählt aus Ethylenmaleinsäureanhydrid und Styrol-Copolymer mit einer Mischung aus Acrylsäure und Methacrylsäure.

2. Zusammensetzung nach Anspruch 1, worin das Virusantigen ein Hundecoronavirusantigen ist.

3. Zusammensetzung nach Anspruch 2, worin das Virusantigen ein avirulentes Antigenprodukt ist, welches hergestellt wird entweder
(a) durch Abschwächung des Hundecoronalebendvirus mittels Passage durch Zellen von Katzenursprung derart, daß das abgeschwächte Lebendvirus bei einer Verabreichung an einen Hund mittels Injektion das Darmepithel selektiv infiziert, oder
(b) durch Inaktivierung eines in Katzen- oder Hundezellen propagierten Hundecoronavirus.

4. Zusammensetzung nach Anspruch 3, worin das avirulente Produkt ein inaktiviertes Hundecoronavirusprodukt mit einem Präinaktivierungstiter von etwa 10.000 bis 10,000,000 Virusteilchen pro Dosis, gemessen durch die FAID₅₀-Methode, ist.

5. Zusammensetzung nach einem der Ansprüche 1 bis 4, worin das Adjuvans Ethylenmaleinsäureanhydrid ist.

## Patentansprüche (Patentansprüche für folgende(n) Vertragsstaat(en): AT)

1. Verfahren zur Herstellung einer Veterinärvakzinezusammensetzung, welches die Vereinigung eines Virusantigens, eines pharmazeutisch akzeptablen Trägers und eines Adjuvans, u.zw. mindestens eines Mitglieds, ausgewählt aus Ethylenmaleinsäureanhydrid und Styrol-Copolymer mit einer Mischung aus Acrylsäure und Methacrylsäure umfaßt.

2. Verfahren nach Anspruch 1, worin das Virusantigen ein Hundecoronavirusantigen ist.

3. Verfahren nach Anspruch 2, worin das Virusantigen ein avirulentes Antigenprodukt ist, welches hergestellt wird entweder
(a) durch Abschwächung des Hundecoronalebendvirus mittels Passage durch Zellen von Katzenursprung derart, daß das abgeschwächte Lebendvirus bei einer Verabreichung an einen Hund mittels Injektion das Darmepithel selektiv infiziert, oder
(b) durch Inaktivierung eines in Katzen- oder Hundezellen propagierten Hundecoronavirus.

4. Verfahren nach Anspruch 3, worin das avirulente Produkt ein inaktiviertes Hundecoronavirusprodukt mit einem Präinaktivierungstiter von etwa 10.000 bis 10,000,000 Virusteilchen pro Dosis, gemessen durch die FAID₅₀-Methode, ist.

5. Verfahren nach einem der Ansprüche 1 bis 4, worin das Adjuvans Ethylenmaleinsäureanhydrid ist.

## Revendications (Revendications pour l'(les) Etat(s) contractant(s) suivant(s): BE, CH, DE, FR, GB, LI, LU, NL, SE)

1. Composition de vaccin vétérinaire comprenant un antigène viral, un excipient pharmaceutiquement acceptable et un adjuvant qui est au moins un membre choisi parmi anhydride éthylène-maléique et un copolymère de styrène avec un mélange d'acide acrylique et d'acide méthacrylique.

2. Composition suivant la revendication 1, dans laquelle l'antigène viral est celui du coronavirus canin.

3. Composition suivant la revendication 2, dans laquelle l'antigène viral est le produit antigénique avirulent produit :
(a) en atténuant le coronavirus canin vivant par passage dans des cellules d'origine féline de façon que lorsqu'il est administré à un chien par injection, le virus vivant atténué affecte sélectivement l'épithélium intestinal, ou bien
(b) en inactivant le coronavirus canin propagé dans des cellules félines ou canines.

4. Composition suivant la revendication 3, dans laquelle le produit avirulent est un produit de coronavirus canin inactivé ayant un titre avant inactivation d'environ 10 000 à 10 000 000 de particules de virus par dose mesuré par le procédé FAID₅₀.

5. Composition suivant l'une quelconque des revendications 1 à 4, dans laquelle l'adjuvant est l'anhydride éthylène-maléique.

## Revendications (Revendications pour l'(les) Etat(s) contractant(s) suivant(s): AT)

1. Procédé de préparation d'une composition de vaccin vétérinaire qui comprend la mise en association d'un antigène viral, d'un excipient pharmaceutiquement acceptable et d'un adjuvant qui est au moins un membre choisi parmi anhydride éthylène-maléique et un copolymère de styrène avec un mélange d'acide acrylique et d'acide méthacrylique.

2. Procédé suivant la revendication 1, dans lequel l'antigène viral est celui du coronavirus canin.

3. Procédé suivant la revendication 2, dans lequel l'antigène viral est le produit avirulent antigénique produit :
(a) en atténuant le coronavirus canin vivant par passage dans des cellules d'origine féline de façon que lorsqu'il est administré à un chien par injection, le virus vivant atténué infecte sélectivement l'épithélium intestinal, ou bien
(b) en inactivant le coronavirus canin propagé dans des cellules félines ou canines.

4. Procédé suivant la revendication 3, dans lequel le produit avirulent est un produit de coronavirus canin inactivé ayant un titre avant inactivation d'environ 10 000 à 10 000 000 de particules de virus par dose mesuré par le procédé FAID₅₀.

5. Procédé suivant l'une quelconque des revendications 1 à 4, dans lequel l'adjuvant est l'anhydride éthylène-maléique.
